# EUROPEAN PATENT SPECIFICATION

(11) **EP 3 606 437 B1**
(45) Date of publication and mention of the grant of the patent: **07.12.2022**
(21) Application number: 18718719.0
(22) Date of filing: 02.04.2018
(51) Int. Cl.: A61B 8/06, A61B 8/08, A61B 5/026, G06T 7/00, G16H 50/30

(54) **METHOD AND APPARATUS FOR PHYSIOLOGICAL FUNCTIONAL PARAMETER DETERMINATION**
VERFAHREN UND VORRICHTUNG ZUR BESTIMMUNG PHYSIOLOGISCHER FUNKTIONELLER PARAMETER
PROCÉDÉ ET APPAREIL DE DÉTERMINATION DE PARAMÈTRE FONCTIONNEL PHYSIOLOGIQUE

(30) Priority: 05.04.2017 EP 17165053
(43) Date of publication of application: 12.02.2020
(73) Proprietor: Koninklijke Philips N.V., 5656 AG Eindhoven (NL)
(72) Inventor: NICKISCH, Hannes, 5656 AE Eindhoven (NL); GRASS, Michael, 5656 AE Eindhoven (NL); HAASE, Christian, 5656 AE Eindhoven (NL); SCHMITT, Holger, 5656 AE Eindhoven (NL)
(74) Representative: Philips Intellectual Property & Standards
(86) International application number: PCT/EP2018/058374
(87) International publication number: WO 2018/185038

(56) References cited:
- EP-A1- 2 633 815
- WO-A1-2013/170053
- WO-A1-2016/001017
- US-A1- 2016 166 209
- US-A1- 2016 364 859

## Description

### FIELD OF THE INVENTION

The present invention relates to an apparatus, a method and a computer program for determining of a physiological functional parameter of a living being.

### BACKGROUND OF THE INVENTION

Functional physiological parameters such as the fractional flow reserve (FFR) are an important predictor of vascular health. They are typically measured invasively. Alternatively, the fluid dynamics within the vascular system can be simulated based on a computed tomography (CT) image of the vascular system, in order to determine the FFR value. However, a patient is exposed to ionizing radiation during computed tomography imaging, which can harm the patient.

EP 2633815 A1 discloses approaches for assessing hemodynamic characteristics of an organ of interest, wherein a fluid dynamics model is provided with data derived from an anatomic imaging modality and with blood flow information derived by ultrasound to determine the desired hemodynamic characteristics.

WO 2013/170053 A1 discloses an ultrasound imaging system using a magnetic linear motor driven ultrasound scanner, with track and hold operation and/or other motion feedback, to scan a two dimensional or three dimensional area of a sample. The scanner is implemented in a low-power and low-bandwidth handheld device and is connected with a remote image processing system that receives raw data and performs full ultrasound image analysis and creation, allowing the handheld device to be used for scanning, preprocessing, and display.

### SUMMARY OF THE INVENTION

It is an object of the present invention to provide an apparatus, a method and a computer program for determining one or more physiological functional parameters, in particular an intravascular pressure gradient or a peripheral FFR, of a living being in a manner being harmless for the health of the living being.

The object is achieved by the apparatus, method and computer program according to the independent claims. All the local anatomical and functional information comprised in ultrasound image data is incorporated into a comprehensive functional patient model allowing to predict physiological functional parameters such as pressure gradients or (peripheral) FFRs without requiring patient data obtained by using ionizing radiation.

In a first aspect of the present invention an apparatus for determining a physiological functional parameter of a living being according to claim 1 is presented, wherein the apparatus comprises:
an image providing unit for providing ultrasound image data and Doppler image data of a vessel structure of the living being;
a registration unit for registering the ultrasound image data and the Doppler image data;
a segmentation unit for segmenting the vessel structure in the ultrasound image data, thereby generating a vessel structure segmentation;
a representation generation unit for generating a representation of the vessel structure based on the vessel structure segmentation;
a flow velocity value determination unit for determining flow velocity values inside a vessel of the vessel structure based on the Doppler image data; and
a physiological functional parameter determination unit for determining the physiological functional parameter of the living being, wherein the physiological functional parameter determination unit is adapted to a) provide a functional parameter determination model defining a functional physiological parameter in dependence of a representation of a vessel structure and flow velocity values inside a vessel of the vessel structure and b) determine the physiological functional parameter by using the functional parameter determination model, the generated representation of the vessel structure and the determined flow velocity values.

The image providing unit provides ultrasound image data and Doppler image data of a vessel structure, wherein the vessel structured covered by the Doppler image data is also covered by the ultrasound image data. The image providing unit may provide the ultrasound image data and the Doppler image data by receiving live image data directly from an ultrasound probe which is positioned over a patient's skin. The image providing unit may also provide the image data from a local memory or a remote server, e.g. image data of a previous session etc. The Doppler image data may be generated from ultrasound Doppler data recorded upon explicit command or automatically with the pure ultrasound image data. The Doppler image data do not have to be provided for each and every position within the ultrasound image data. A sonographer may activate Doppler image data taking for instance by pressing a particular button and thus control the respective regions in which ultrasound Doppler image data is taken in addition to pure ultrasound image data.

The ultrasound image includes several ultrasound images for several times and/or for several phases, especially for different cardiac phases. Correspondingly, also the Doppler image data includes several Doppler images for different times and/or for different phases. The registration unit is adapted to temporally and spatially align the ultrasound image data and the Doppler image data. The registration unit may use information extracted from the image data itself and/or external data, like timestamps (relative to the cardiac cycle) and position data if available. The registration unit may for instance extract information from the image data by using local image features such as speckles, vector flow images or anatomy which may guide the alignment via a similarity measure e.g. correlation. As pointed out herein above images taken for one and the same position of an ultrasound probe over the skin of a living being do not necessarily show a static image. Across a cardiac cycle, the vessel structure might show some movements. Thus, temporal and spatial alignment may be linked. This is of particular importance when stitching images together that cover different areas with a certain spatial overlay. The temporal alignment within the cardiac cycle might impact the spatial matching, in particular when performed automatically, e.g. error margins might be larger for two images taken at different times of the cardiac cycle than for images taken at the same time of the cardiac cycle. Alternatively or in addition, the registration unit may also exploit data provided by hardware support, e.g. a set of stationary external tracking cameras followed by image processing, a tracking device specifically designed for the ultrasound probe or a transducer-internal tracking device (gyrometer or optoelectronic sensor) which can specify the absolute and/or relative position of an ultrasound probe for each image up to a certain accuracy.

The segmentation unit segments the ultrasound image data to identify the vessel structure. Image segmentation may be performed using a variety of techniques which might be used standalone or in combination to identify meaningful structures of interest in the image data. WO 2016/156446 A1 discloses an ultrasound system and method for identifying vessel structures.

For the present invention, it is sufficient to obtain local information, preferably 3-D information, a holistic global 3-D model is not required. Optionally, the segmentation unit might use information from the Doppler image data to classify blood pools (areas indicating moving blood) from the surrounding static tissue. Smoothing can preferentially be used in order to compensate for local imaging artifacts. The segmentation of a vessel and the vessel wall can further be improved by edge detection algorithms applied to the ultrasound image data. If the full cross-section of the vessel is not visible in the ultrasound image data, local peak flow velocity values obtained from the Doppler image data can be used to derive the corresponding local cross-sectional area of a vessel and this might further be used for the segmentation.

The representation generation unit generates a representation from the segmented vessel structure. The representation is preferentially a 3D-representation of the vessel structure including at least the major vessels contained in the image data.

A flow velocity determination unit is used to determine flow velocity values from the Doppler image data. Preferentially, static average flow velocity values are determined as the average over a local neighborhood. Depending on the temporal resolution of the Doppler image data, flow velocity values may be determined as values averaged over a cardiac cycle. Alternatively, the flow velocity values may also be determined as a function of time, e.g. over a cardiac cycle, as a flow velocity profile. If the Doppler image data provides input data for more than one cardiac cycle, the flow velocity profile may also provide average flow velocity values for the respective times of the cardiac cycle.

Based on the determined flow velocity values and the representation, the physiological functional parameter determination unit determines the physiological functional parameters using the physiological functional parameter determination model that defines a functional physiological parameter in dependence of a vessel structure representation and flow velocities inside a vessel of the vessel structure. The physiological functional parameter determination model is preferentially a reduced-order functional model and the flow velocity values and the representation are provided as boundary conditions to personalize the model for the living being that is being examined.

The physiological functional parameter is preferentially the intravascular pressure gradient or the (peripheral) fractional flow reserve ((p)FFR). The FFR is commonly used to describe coronary arteries, where the FFR is defined as the ratio of the intravascular pressure after (distal to) a stenosis relative to the pressure of the aorta, e.g. Pd/Pa. Thus, the FFR is an absolute number. Peripheral FFR (p-FFR) is a recent attempt to establish a similar functional index for the peripheral arteries, as suggested inter alia by Issam Koleilat, et al. in "A Novel Simple Technique Using Hyperemia to Enhance Pressure Gradient Measurement of the Lower Extremity During Peripheral Intervention", VASCULAR DISEASE MANAGEMENT 2015; 12(9):E166-E172. p-FFR is defined by the ratio of the intravascular pressure after (distal to) a stenosis Pd relative to the pressure of a major artery Pa. The pressure gradient is simply the difference between Pa and Pd measured in pressure per length unit.

The functional parameter determination model is preferentially a reduced order functional model, in particular a lumped parameter model, wherein the representation of the vessel structure and the flow velocity values or value profiles extracted from the Doppler image data are used as boundary conditions. The use of lumped parameter models for physiological functional parameter determination is discussed inter alia in "Learning patient-specific lumped models for interactive coronary blood flow simulations" by Hannes Nickisch et al., Medical Image Computing and Computer-Assisted Intervention-MICCAI 2015 (pp. 433-441), Springer International Publishing (2015).

Since reduced order functional model predictions, in particular lumped parameter model predictions, can be computed extremely fast, the results may be provided to sonographers and/or physicians in real-time or at least near-real-time and thus support the diagnosis and treatment of for instance arteriosclerosis.

Preferentially, the apparatus may output the representation as well as the determined physiological functional parameter via a display/monitor wherein the physiological functional parameter may be indicated as color code in the representation of the vessel structure. The display/monitor may be integrally formed with the apparatus or may be an external component connected either wired or wirelessly with the apparatus. Thus, a sonographer conducting the ultrasound probe examination and the person, for instance a physician, viewing the physiological functional parameter and the representation do not have to be the same. They do not even have to be in the same room. A physician might review the examination results from an entirely different location in real-time.

In an embodiment the Doppler image data are first Doppler image data of a first portion of the vessel structure and the determined flow velocity values are first flow velocity values inside a vessel of the first portion of the vessel structure, wherein
the image providing unit is configured to provide second Doppler image data of a second portion of the vessel structure;
the registration unit is configured to register the second Doppler image data with the ultrasound image;
the flow velocity value determination unit is configured to determine second flow velocity values inside a vessel of the second portion of the vessel structure based on the second Doppler image data; and
the physiological functional parameter determination unit is configured to determine the physiological functional parameter using the provided functional parameter determination model, the generated representation of the vessel structure and the determined first and second flow velocity values.

In this embodiment the physiological functional parameter determination can be updated whenever new Doppler image data are provided. The ultrasound image data provided and used to segment the vessel structure may cover several vessels of a vessel structure. Doppler images may be provided for respective positions within the ultrasound image data to determine respective flow velocity values for vessels at these positions. The first Doppler image data may cover a first vessel of the vessel structure covered by the ultrasound image, the second Doppler image data may cover a second vessel of the vessel structure or the first vessel at another location within the vessel structure. The flow velocity values may be fed to the physiological functional parameter determination unit to update the physiological functional parameter determination. The additional flow velocity values may be provided as additional boundary conditions and thus provide a revised physiological functional parameter determination of the entire vessel structure. Newly added Doppler image data may thus also impact the determination of a previously determined physiological functional parameter of another portion of the vessel structure, since the entire model is updated.

In a further embodiment the ultrasound image data are a first ultrasound image data covering a first part of the vessel structure and the generated vessel structure segmentation is a first vessel structure segmentation, wherein
the image providing unit is configured to provide second ultrasound image data of the vessel structure covering a second part of the vessel structure, wherein the second part differs at least partially from the first part;
the registration unit is configured to register the second ultrasound image data with the first ultrasound image data;
the segmentation unit is configured to segment the vessel structure in the second ultrasound image data thereby generating a second vessel structure segmentation; the representation generation unit is configured to generate the representation of the vessel structure based on the first vessel structure segmentation and based on the second vessel structure segmentation.

In this embodiment, the representation of the vessel structure is updated whenever new ultrasound image data are provided. Preferentially, the first and second ultrasound image data have a certain overlap. Then, characteristic features in the overlaying image parts can be used to stitch the image data together. If there is no overlap between the ultrasound image data, e.g. if the ultrasound probe has been discontinuously moved over the skin of the living being or had not continuously taken data, external position information might be provided in order to properly register the first and second ultrasound image data. The representation generation unit updates the representation based on the vessel structure segmented in the first and second ultrasound image data. The additional input can thus be used to update the representation of the vessel structure in real-time. Image registration and segmentation might influence one another and the respective steps may be performed in an iterative manner. The apparatus is preferentially adapted to display the representation of the vessel structure examined with the ultrasound probe and thus provide a live-growing representation whenever a new ultrasound image is provided. Furthermore, since the representation is used as input for the physiological functional parameter determination unit, e.g. as boundary condition of a reduced-order functional model, the further ultrasound image data may also impact the physiological functional parameter determination.

Usually the movement of an ultrasound probe along the skin of a living being will provide both further ultrasound images which extend the coverage area of the vessel structure and further Doppler images which provide further flow velocity measurements. Along with the live-growing representation, e.g. a 3D-model of the vessel structure, the functional parameter determination model is further updated with every new information obtained, e.g. a lumped parameter model is constantly updated with further boundary conditions obtained from the extended representation of the vessel structure as well as the flow velocity values from the Doppler images. Further input data may increase the accuracy of an existing representation as well as the lumped parameter model. Thus, the update of the representation as well as the lumped parameter model may also impact previously determined values of the vessel structure.

In a second aspect of the present invention a method for determining a physiological functional parameter of a living being according to claim 10 is presented, the method comprising:
providing ultrasound image data and Doppler image data of a vessel structure of the living being;
registering the ultrasound image data and the Doppler image data temporally and spatially, thereby obtaining registered image data;
segmenting the vessel structure in the registered image data;
generating a representation of the vessel structure based on the segmented vessel structure, thereby generating a vessel structure segmentation;
determining flow velocity values inside a vessel of the vessel structure based on the Doppler image data;
providing a functional parameter determination model defining a functional physiological parameter in dependence of a representation of a vessel structure and flow velocity values inside a vessel of the vessel structure;
determining the physiological functional parameter of the living being by using the functional parameter determination model, the generated representation of the vessel structure and the determined flow velocity values.

In an embodiment the Doppler image data are first Doppler image data of a first portion of the vessel structure and the determined flow velocity values are first flow velocity values inside a vessel of the first portion of the vessel structure, and the method further comprises
providing second Doppler image data of a second portion of the vessel structure;
registering the second ultrasound Doppler image data with the first ultrasound image data;
determining second flow velocity values inside a vessel of the second portion of the vessel structure based on the second Doppler image data; and
determining the physiological functional parameter using the provided functional parameter determination model, the generated representation of the vessel structure and the first and second flow velocity values.

In an embodiment the ultrasound image data are first ultrasound image data covering a first part of the vessel structure, wherein the generated vessel structure segmentation is a first vessel structure segmentation and wherein the method further comprises:
providing second ultrasound image data of the vessel structure covering a second part of the vessel structure, wherein the second part differs at least partially from the first part;
registering the second ultrasound image data with the first ultrasound image data;
segmenting the vessel structure in the second ultrasound image data, thereby generating a second vessel structure segmentation;
generating the representation of the vessel structure based on the first vessel structure segmentation and the second vessel structure segmentation.

In a third aspect of the present invention a computer program executable in a processing unit of an apparatus as defined in the first aspect of the invention is presented, the computer program comprising program code means for causing the processing unit to carry out a method as defined in the second aspect of the invention when the computer program is executed in the processing unit.

It shall be understood that the apparatus for determining a physiological functional parameter of a living being according to the first aspect of the invention, the method for determining a physiological functional parameter of a living being according to the second aspect of the invention, and the computer program for determining a physiological functional parameter of a living being of the third aspect of the invention have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily shows an embodiment of an apparatus for determining a physiological functional parameter of a living being;
Fig. 2 schematically and exemplarily shows an embodiment of a method for determining a physiological functional parameter of a living being;
Fig. 3 schematically and exemplarily illustrates a vessel structure derivable with an embodiment of an apparatus or method for determining a physiological functional parameter of a living being;
Fig. 4 schematically and exemplarily shows a lumped parameter model usable to determine a physiological functional parameter a living being; and
Fig. 5 schematically and exemplarily illustrates a measured flow velocity along a vessel and a derived cross-sectional area along the vessel.

### DETAILED DESCRIPTION OF EMBODIMENTS

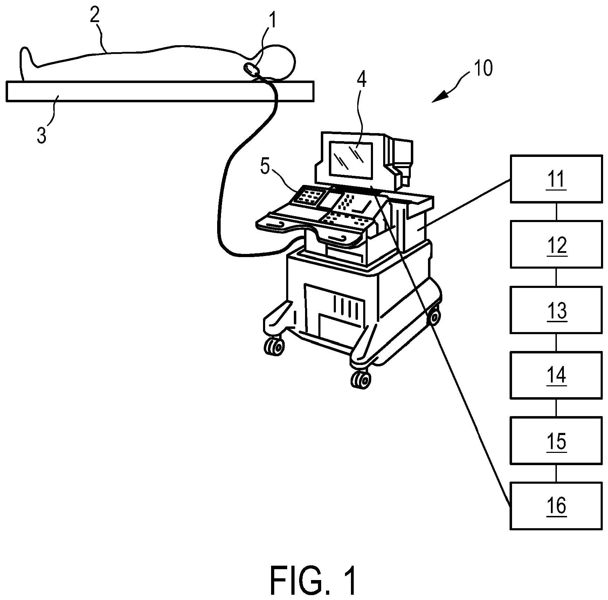

Fig. 1 schematically and exemplarily shows an embodiment of an apparatus 10 for determining a physiological functional parameter of a living being. In this embodiment, the apparatus 10 comprises an ultrasound probe 1 for providing ultrasound image data when being traversed over the skin of a person 2 lying on support means 3. The ultrasound probe 1 can provide both common ultrasound image data as well as Doppler image data wherein the operation mode may be changed manually, e.g. upon pressing a particular button, or automatically, e.g. on a predetermined periodical basis or whenever the probe is moved by a predetermined distance. The ultrasound probe may comprise a tracking device (not shown) specifically designed for the ultrasound probe or a transducer-internal tracking device (gyrometer) (not shown). In order to determine the ultrasound probe's position other hardware may be used, e.g. a set of external tracking cameras followed by image processing, or specific distance sensors for instance integrated in the support means 3. The apparatus 10 further comprises input means 5 which allow the input of specific commands by a user, like start and stop, relevant patient data and/or position data which may optionally be attached to the image data captured with the ultrasound probe 1 for further processing. The ultrasound probe 1 is communicatively coupled to the apparatus 10, either wired or wirelessly. Thus, the ultrasound probe 1 may be within the same room as the apparatus 10 but the apparatus 10 may also be at a completely different location and the ultrasound probe 1 is merely connected to the apparatus 10 via the Internet.

The apparatus 10 has an image providing unit 11 to provide data, preferentially live from the ultrasound probe 1. Alternatively or in addition, the image providing unit 11 may provide images from memory, either stored locally at the apparatus or on a remote server. For instance, in case ultrasound images have already been taken from the person 2, already existing data may be loaded by the image providing unit 11. The image providing unit 11 may also provide partially existing data from memory and add for instance Doppler images for certain positions within the existing pure ultrasound images.

The apparatus 10 further comprises a registration unit 12 for registering the images provided by the image providing unit. With regard to the spatial alignment different sets of data are transformed into one coordinate system. If the ultrasound probe 1 is for instance moved along the skin of the person 2, and a first and second ultrasound image cover a common area of the vessel structure, then similarities in the image data can be used to overlay the images and stitch them together.

However, ultrasound images, in particular Doppler images, may differ not only when the ultrasound probe 1 is moved along the skin of the person 2 and thus the spatial information is changed. The blood flow inside a vessel also fluctuates within a cardiac cycle and an artery or vein might slightly vary in position and/or cross section over a cardiac cycle. If the image data is thus taken once at the peak of a cardiac cycle and once on the lowest point, automatic detection of similarities might require larger error margins to match respective image contours or specific features. Thus, temporal information preferably associated with the cardiac cycle might be used by the registration unit to stitch images together which cover respective first and second parts of a vessel structure having a certain overlay. Furthermore, absolute or relative position data of the ultrasound probe 1 can also be provided to the registration unit 12 to provide a consistent mosaic of ultrasound images and additional Doppler images. Usually ultrasound images are provided in grey scale while Doppler images are overlaid in a color scale, e.g. from blue to red. Usually blue color indicates a flow away from the ultrasound probe 1, and red color indicates a flow towards the ultrasound probe 1. Depending on the chosen resolution, the color might change during a cardiac cycle. When a temporal resolution is required which is sensitive to the cardiac cycle, respective flow profiles across a cardiac cycle are generated. Doppler image data may then comprise a set of Doppler images for respective times within the cardiac cycle. They may be generated from data over one or more cardiac cycles. The flow velocities determined for vessels in the Doppler images may then be provided as flow velocity profiles across a cardiac cycle. If such a temporal resolution is not necessary, static average flow velocity values are determined from the Doppler image data.

Optionally, 3-D ultrasound transducers may be used to gather additional information for the rigid image registration. For instance, in conjunction with an optoelectric sensor (as used in a computer mouse) the trajectory of the transducer can be traced. In order to simplify the image registration, restrictions on the possible trajectories may be imposed by means of a user manual. Registration can be done in the simplest instance by matching image locations using squared distance or correlation measures. Given the information from external or internal tracking devices, the latter could be used to fine-tune the registration.

The apparatus 10 further comprises a segmentation unit 13 for segmenting a vessel structure identified in ultrasound image data. If there is more than one ultrasound image provided by the image providing unit 11, the segmentation unit may also segment a vessel structure from first and second ultrasound images stitched by the registration unit 12. The segmentation unit 13 may use static image features such as the edges of the lumen to segment the vessel structure and/or use dynamic features derived from the Doppler images which for instance indicates flowing blood and thus help to determine the inside of a vessel. The surrounding tissue of a vessel is rather static. The segmentation unit 13 may also allow or request user input to improve or initiate image segmentation. Furthermore, the segmentation unit 13 may smooth the image data provided to compensate for local imaging artifacts. The segmentation unit 13 may also be provided with external input provided by a user via the input means 5 in order to guide the segmentation (a priori) or to correct the segmentation (a posteriori). In order to support the user's assessment, overlays of ultrasound images and the deduced segmentation could be displayed on a display unit 4.

The vessel structure segmented from the aligned ultrasound images is used by a representation generation unit 14 to generate a common representation 200 of the corresponding vessel structure of interest, preferably as a 3D-model. This representation 200 is continuously updated whenever new input data is provided to the segmentation unit, e.g. whenever the ultrasound probe 1 is moved along the skin of the person 2.

Along with the live-growing representation, a physiological functional parameter determination model, e.g. a lumped parameter model 210, is generated and continuously updated which allows to estimate for instance pressure gradients for vessels inside the vessel structure in real-time given a set of suitable boundary conditions. The flow velocity inside a vessel can be determined from the Doppler images and can be used as boundary condition for the lumped parameter model 210. Therefore, the apparatus 10 further comprises a flow velocity determination unit 15 for determining average flow velocities or flow velocity profiles which are provided as boundary conditions to the lumped parameter model 210. Since the Doppler data may only be provided for a portion of the vessel structure, the determination of flow velocities is restricted to these portions. They will preferably capture the most relevant arteries and/or veins in the vessel structure of interest. The apparatus 10 further comprises a physiological functional parameter determination unit 16 for determining pressure gradients or peripheral FFR values using the lumped parameter model 210. Since lumped model predictions can be computed extremely fast, the apparatus 10 may provide real-time or at least near-real-time feedback in clinical practice and thus support sonographers and/or physicians in the diagnosis and treatment of for instance arteriosclerosis. The feedback may be presented in form of the representation 200 which is preferentially color coded according to the determined pressure gradients at the display unit 4 of the apparatus 10 in Fig. 1. Alternatively or in addition, the apparatus 10 may also provide the output to a remote display connected either wired or wirelessly with the apparatus. This way, the person conducting the examination of the person 2 and the person assessing the determined data do not have to be the same. The latter person does not even have to be within the same room. The apparatus 10 may provide the data output for storage, either locally, on hard drive or removable storage, or remote, e.g. at a server or cloud.

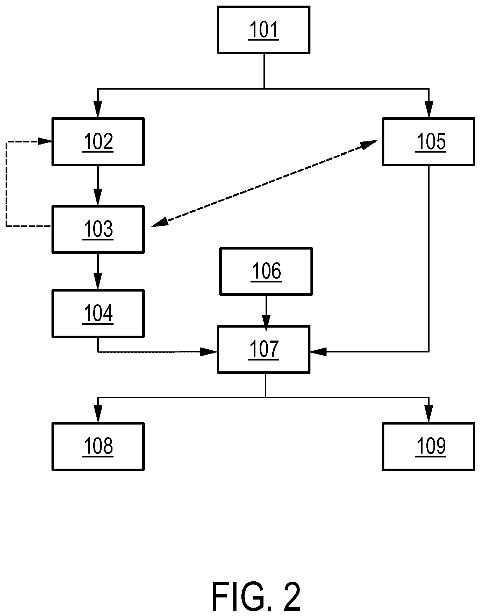

In the following an embodiment of a method for determining a physiological functional parameter of a living being will be exemplarily described with reference to a flow chart shown in Fig. 2.

In step 101 first ultrasound image data covering a first part of the vessel structure of interest are provided, preferentially obtained from an ultrasound probe 1. Furthermore, Doppler image data are provided covering at least a portion of the vessel structure covered by the ultrasound image data. The ultrasound image data are then registered in step 102 with the Doppler image data. In step 103 a vessel structure is segmented from the image data. Step 103 may be performed parallel, subsequent or iteratively with step 102. In case only the ultrasound image data are used for the segmentation, step 103 may be performed independent of step 102. The segmentation comprises basic identification of image structures, such as contours and edges as well as high-level image segmentation specific to structures and patterns typical for the vessel structures. The segmentation step 103 might use further input extracted from the Doppler image data. In that case the previous registration in step 102 is required to merge the information from both image data. In step 104, a representation of vessel structure of interest is generated from the segmented image data. This representation is preferentially a 3D-representation of the main vessels of a vessel structure of interest.

In step 105 flow velocity values are determined from the Doppler image data for respective positions within one or more vessels of a vessel structure. Depending on the temporal resolution average flow velocity values, or flow velocity profiles across a cardiac cycle can be determined. This step may be performed simultaneously to the steps 102 and 103. The Doppler image data may optionally be used for segmentation as indicated by the dashed line between step 105 and step 103. The Doppler image data allow identification of areas with moving blood, in general inside an artery or vein, and the surrounding tissue, which is static and thus does not show the Doppler effect.

In step 107 a physiological functional parameter, e.g. the vascular pressure gradient or the (peripheral) FFR, may be determined for vessels in the representation of the vessel structure using a reduced-order functional model based on determined flow velocities, and the representation of the vessel structure provided in step 106. The determination may use a lumped parameter model wherein the average values of flow velocity, or the flow velocity profiles as well as the representation of the physical vessel structure are used as boundary conditions.

The method may comprise a further step 108 of outputting the representation, preferentially as 3D-model, together with the determined physiological functional parameter values which might be presented in a color-coded manner, wherein specific colors are assigned to predetermined thresholds values of the physiological functional parameter to ease a fast assessment and assist a physician in the diagnosis.

Whenever the ultrasound probe is moved, the method starts again at step 101. Additional image data are provided and registered using internal image information like characteristic patterns as well as external data, cardiac cycle data, position data of the ultrasound probe, etc. The method may also only be fed with further Doppler image data in a region already captured by pure ultrasound image data. In that case, the method continues with step 105 and from there either to step 103, where the Doppler image data are used for segmentation purposes and/or to step 107 to update and extend the lumped parameter model accordingly and determine the physiological functional parameter for the (extended) vessel structure based on the further flow velocity measurements derived from the further Doppler image data. Besides outputting the 3D-representation and the determined physiological functional parameters, the output may also be stored in step 109 in a local or remote storage device, such as a hard drive, optical disc, removable storage medium (USB stick), or on a remote server. Furthermore, the step 108 may comprise transmission of the data via a network to a remote display, such that the person viewing the live-growing anatomical and functional representation does not have to be in the same room as the person 2 examined with the ultrasound probe 1.

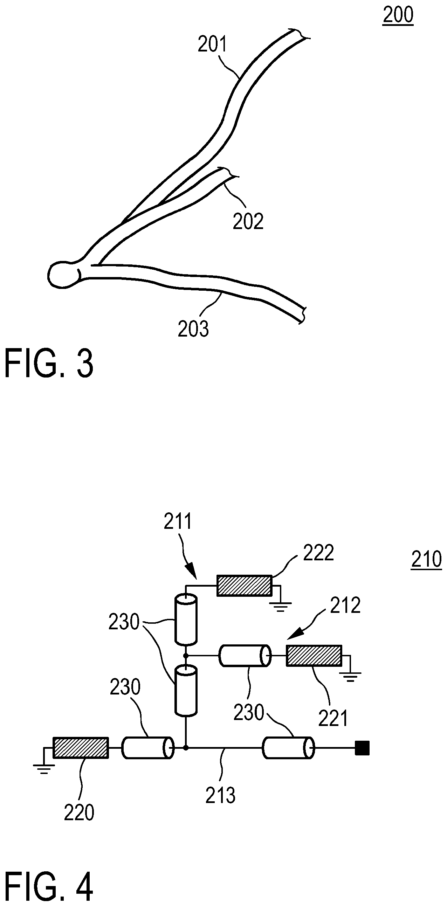

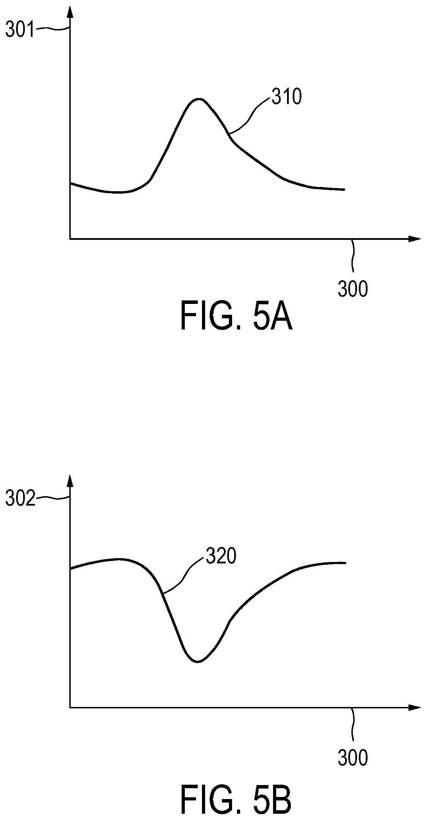

Fig. 3 schematically and exemplarily illustrates a vessel structure derivable from the ultrasound image data. The vessel structure may be determined using static image features such as the edges of the lumen or dynamic features derivable from Doppler image data which allow to distinguish moving areas, e.g. flowing blood instance areas, e.g. tissue. Additionally or in cases where the full cross section of a vessel is not visible in the ultrasound image data, the local peak flow velocity values 301 derivable from the Doppler image data can be used to derive the local cross sectional area 302 (CSA) as indicated in Figs. 5A and 5B and thus might help to segment the vessel structure.

Fig. 5A shows the flow velocity 301 over the vessel length 300. The resulting curve 310 has the opposite distribution as curve 320, showing the cross sectional area 302 over the vessel length 300. Here a constant blood volume is assumed. Estimating or measuring outflow can further refine this calculation.

The extracted anatomical vessel structure 200 comprising branches 201, 202 and 203 may then be used together with the flow velocity measurements as boundary condition for a reduced-order functional model i.e. a lumped parameter model 210.

Fig. 4 schematically and exemplarily shows a lumped parameter model 210 with three branches 211, 212 and 213 corresponding to branches 201 to 203 of the vessel structure representation 200. The lumped parameter model comprises n = 8 elements and m = 3 nodes including ground. The black boxes 220, 221, 222 indicate inflow and outflow boundary conditions. The white tubes 230 representing tree segment transfer functions are composed of a series of linear and nonlinear resistance elements reflecting both the local vessel geometry and hydraulic effects. Starting from the tree representation shown in Fig. 3, a circuit with two macroscopic component types is set up: nonlinear vessel segment resistors 230 and boundary conditions 220, 221, 222. The boundary condition may be a pressure or flow source driving the network; but any (lumped) boundary condition driving a conventional finite element model can be used here. Methods how to translate the local geometry of the vessel (radius, perimeter, cross-sectional area) into parameters of the nonlinear resistor are well known and disclosed, for instance, in the article "Learning patient-specific lumped models for interactive coronary blood flow simulations" by Hannes Nickisch et al. cited herein above.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. An apparatus (10) for determining a physiological functional parameter of a living being (2), the apparatus (10) comprising:
an image providing unit (11) for providing ultrasound image data and Doppler image data of a vessel structure of the living being (2);
a registration unit (12) configured to temporally and spatially align the ultrasound image data and the Doppler image data for registering the ultrasound image data and the Doppler image data to obtain a registered image data;
a segmentation unit (13) for segmenting the vessel structure in the registered image data, thereby generating a vessel structure segmentation;
a representation generation unit (14) for generating a representation (200) of the vessel structure based on the vessel structure segmentation;
a flow velocity determination unit (15) for determining flow velocity values inside a vessel of the vessel structure based on the Doppler image data; and
a physiological functional parameter determination unit (16) for determining the physiological functional parameter of the living being (2), wherein the physiological functional parameter determination unit is adapted to a) provide a functional parameter determination model (210) defining a functional physiological parameter in dependence of a representation of a vessel structure and flow velocity values inside a vessel of the vessel structure, and b) determine the physiological functional parameter by using the functional parameter determination model, the generated representation of the vessel structure and the determined flow velocity values.

2. The apparatus (10) according to claim 1, wherein
the Doppler image data are first Doppler image data of a first portion of the vessel structure and the determined flow velocity values are first flow velocity values inside a vessel of the first portion of the vessel structure;
the image providing unit (11) is configured to provide second Doppler image data of a second portion of the vessel structure;
the registration unit (12) is configured to register the second Doppler image data with the ultrasound image data;
the flow velocity value determination unit (15) is configured to determine second flow velocity values inside a vessel of the second portion of the vessel structure based on the second Doppler image data; and
the physiological functional parameter determination unit (16) is configured to determine the physiological functional parameter using the provided functional parameter determination model (210), the generated representation of the vessel structure and the determined first and second flow velocity values.

3. The apparatus (10) according to claim 1, wherein the ultrasound image data are first ultrasound image data covering a first part of the vessel structure, wherein the generated vessel structure segmentation is a first vessel structure segmentation and wherein
the image providing unit (11) is further configured to provide second ultrasound image data of the vessel structure covering a second part of the vessel structure, wherein the second part differs at least partially from the first part;
the registration unit (12) is configured to register the second ultrasound image data with the first ultrasound image data;
the segmentation unit (13) is configured to segment the vessel structure in the second ultrasound image data, thereby generating a second vessel structure segmentation;
the representation generation unit (14) is configured to generate the representation (200) of the vessel structure based on the first vessel structure segmentation and based on the second vessel structure segmentation.

4. The apparatus (10) according to claim 1, wherein the registration unit (12) comprises a position detection unit providing position data of an ultrasound probe (1) used to generate the ultrasound image data and the Doppler image data, wherein the registration unit (12) is adapted to use the position data for registering the ultrasound image data and the Doppler image data.

5. The apparatus (10) according to claim 1, wherein the segmentation unit (13) is configured to identify moving and static areas in the Doppler image data and to segment the vessel structure based at least in part on the identified moving and static areas.

6. The apparatus (10) according to claim 5, wherein the segmentation unit (13) is configured to determine local peak flow values for a vessel of the vessel structure in the moving areas of the Doppler image data, to determine cross-sectional areas of the vessel based on the local peak flow values and to segment the vessel structure in the registered image data by using the cross-sectional areas.

7. The apparatus (10) according to claim 5, wherein the segmentation unit (13) is further configured to segment the vessel structure in the registered image data by using a lumen edge detection algorithm.

8. The apparatus (10) according to claim 1, wherein the physiological functional parameter is a vascular pressure gradient or a peripheral fractional flow reserve.

9. The apparatus (10) according to claim 1, wherein the functional parameter determination model is a reduced order functional model.

10. A method for determining a physiological functional parameter of a living being (2), the method comprising:
providing (101) ultrasound image data and Doppler image data of a vessel structure of the living being (2) with an image providing unit (11);
registering (102) the ultrasound image data and the Doppler image data temporally and spatially, thereby obtaining registered image data;
segmenting (103) the vessel structure in the registered image data;
generating (104) a representation (200) of the vessel structure based on the segmented vessel structure, thereby generating a vessel structure segmentation;
determining (105) flow velocity values inside a vessel of the vessel structure based on the Doppler image data;
providing (106) a functional parameter determination model (210) defining a functional physiological parameter in dependence of a representation of a vessel structure and flow velocity values inside a vessel of the vessel structure;
determining (107) the physiological functional parameter of the living being (2) by using the functional parameter determination model (210), the generated representation of the vessel structure and the determined flow velocity values.

11. The method according to claim 10, wherein the Doppler image data are first Doppler image data of a first portion of the vessel structure and the determined flow velocity values are first flow velocity values inside a vessel of the first portion of the vessel structure, the method further comprising:
providing (101) second Doppler image data of a second portion of the vessel structure;
registering (102) the second Doppler image data with the ultrasound image data;
determining (104) second flow velocity values inside a vessel of the second portion of the vessel structure based on the second Doppler image data; and
determining (105) the physiological functional parameter using the provided model (210), the generated representation of the vessel structure and the first and second flow velocity values.

12. The method according to claim 10, wherein the ultrasound image data are first ultrasound image data covering a first part of the vessel structure, wherein the generated vessel structure segmentation is a first vessel structure segmentation and wherein the method further comprises:
providing second ultrasound image data of the vessel structure covering a second part of the vessel structure, wherein the second part differs at least partially from the first part;
segmenting the vessel structure in the second ultrasound image data, thereby generating a second vessel structure segmentation;
generating the representation (200) of the vessel structure based on the first vessel structure segmentation and the second vessel structure segmentation.

13. A computer program for determining a physiological functional parameter of a living being (2) executable in a processing unit of an apparatus (10) as defined in claim 1, the computer program comprising program code means for causing the processing unit to carry out a method as defined in claim 10 when the computer program is executed in the processing unit.

## Patentansprüche

1. Apparat (10) zur Bestimmung eines physiologischen Funktionsparameters eines Lebewesens (2), wobei der Apparat (10) Folgendes umfasst:
eine Bildbereitstellungseinheit (11) zur Bereitstellung von Ultraschallbilddaten und Dopplerbilddaten einer Gefäßstruktur des Lebewesens (2);
eine Registrierungseinheit (12), die so konfiguriert ist, dass sie die Ultraschallbilddaten und die Dopplerbilddaten zeitlich und räumlich ausrichtet, um die Ultraschallbilddaten und die Dopplerbilddaten zu registrieren, um registrierte Bilddaten zu erhalten;
eine Segmentierungseinheit (13) zum Segmentieren der Gefäßstruktur in den registrierten Bilddaten, wodurch eine Segmentierung der Gefäßstruktur erzeugt wird;
eine Darstellungserzeugungseinheit (14) zum Erzeugen einer Darstellung (200) der Gefäßstruktur auf der Grundlage der Segmentierung der Gefäßstruktur;
eine Strömungsgeschwindigkeits-Bestimmungseinheit (15) zur Bestimmung von Strömungsgeschwindigkeitswerten innerhalb eines Gefäßes der Gefäßstruktur auf der Grundlage der Doppler-Bilddaten; und
eine physiologische Funktionsparameter-Bestimmungseinheit (16) zum Bestimmen des physiologischen Funktionsparameters des Lebewesens (2), wobei die physiologische Funktionsparameter-Bestimmungseinheit ausgebildet ist, um a) ein Funktionsparameter-Bestimmungsmodell (210) bereitzustellen, das einen funktionalen physiologischen Parameter in Abhängigkeit von einer Darstellung einer Gefäßstruktur und von Strömungsgeschwindigkeitswerten innerhalb eines Gefäßes der Gefäßstruktur definiert, und b) den physiologischen Funktionsparameter unter Verwendung des Funktionsparameter-Bestimmungsmodells, der erzeugten Darstellung der Gefäßstruktur und der bestimmten Strömungsgeschwindigkeitswerte zu bestimmen.

2. Apparat (10) nach Anspruch 1, wobei
die Dopplerbilddaten erste Dopplerbilddaten eines ersten Teils der Gefäßstruktur sind und die ermittelten Strömungsgeschwindigkeitswerte erste Strömungsgeschwindigkeitswerte innerhalb eines Gefäßes des ersten Teils der Gefäßstruktur sind;
die Bildbereitstellungseinheit (11) so konfiguriert ist,
dass sie zweite Dopplerbilddaten eines zweiten Abschnitts der Gefäßstruktur bereitstellt;
die Registrierungseinheit (12) so konfiguriert ist, dass sie die zweiten
Dopplerbilddaten mit den Ultraschallbilddaten registriert;
die Strömungsgeschwindigkeitswert-Bestimmungseinheit (15) konfiguriert ist, um zweite Strömungsgeschwindigkeitswerte innerhalb eines Gefäßes des zweiten Abschnitts der Gefäßstruktur basierend auf den zweiten Dopplerbilddaten zu bestimmen; und
die physiologische Funktionsparameter-Bestimmungseinheit (16) konfiguriert ist, um den physiologischen Funktionsparameter unter Verwendung des bereitgestellten Funktionsparameter-Bestimmungsmodells (210), der erzeugten Darstellung der Gefäßstruktur und der bestimmten ersten und zweiten Strömungsgeschwindigkeitswerte zu bestimmen.

3. Apparat (10) nach Anspruch 1, wobei die Ultraschallbilddaten erste Ultraschallbilddaten sind, die einen ersten Teil der Gefäßstruktur abdecken, wobei die erzeugte Gefäßstruktursegmentierung eine erste Gefäßstruktursegmentierung ist und wobei die Bildbereitstellungseinheit (11) ferner so konfiguriert ist, dass sie zweite Ultraschallbilddaten der Gefäßstruktur bereitstellt, die einen zweiten Teil der Gefäßstruktur abdecken, wobei sich der zweite Teil zumindest teilweise von dem ersten Teil unterscheidet;
die Registrierungseinheit (12) so konfiguriert ist, dass sie die zweiten Ultraschallbilddaten mit den ersten Ultraschallbilddaten registriert;
die Segmentierungseinheit (13) so konfiguriert ist, dass sie die Gefäßstruktur in den zweiten Ultraschallbilddaten segmentiert, wodurch eine zweite Gefäßstruktursegmentierung erzeugt wird;
die Darstellungserzeugungseinheit (14) konfiguriert ist, um die Darstellung (200) der Gefäßstruktur auf der Grundlage der ersten Gefäßstruktursegmentierung und auf der Grundlage der zweiten Gefäßstruktursegmentierung zu erzeugen.

4. Apparat (10) nach Anspruch 1, wobei die Registrierungseinheit (12) eine Positionserfassungseinheit umfasst, die Positionsdaten einer Ultraschallsonde (1) bereitstellt, die verwendet wird, um die Ultraschallbilddaten und die Dopplerbilddaten zu erzeugen, wobei die Registrierungseinheit (12) angepasst ist, um die Positionsdaten zum Registrieren der Ultraschallbilddaten und der Dopplerbilddaten zu verwenden.

5. Apparat (10) nach Anspruch 1, wobei die Segmentierungseinheit (13) so konfiguriert ist, dass sie bewegte und statische Bereiche in den Dopplerbilddaten identifiziert und die Gefäßstruktur zumindest teilweise auf der Grundlage der identifizierten bewegten und statischen Bereiche segmentiert.

6. Apparat (10) nach Anspruch 5, wobei die Segmentierungseinheit (13) konfiguriert ist, um lokale Spitzenflusswerte für ein Gefäß der Gefäßstruktur in den sich bewegenden Bereichen der Dopplerbilddaten zu bestimmen, um Querschnittsflächen des Gefäßes basierend auf den lokalen Spitzenflusswerten zu bestimmen und um die Gefäßstruktur in den registrierten Bilddaten unter Verwendung der Querschnittsflächen zu segmentieren.

7. Apparat (10) nach Anspruch 5, wobei die Segmentierungseinheit (13) ferner so konfiguriert ist, dass sie die Gefäßstruktur in den registrierten Bilddaten unter Verwendung eines Algorithmus zur Lumenranderkennung segmentiert.

8. Apparat (10) nach Anspruch 1, wobei der physiologische Funktionsparameter ein vaskulärer Druckgradient oder eine periphere fraktionale Flussreserve ist.

9. Apparat (10) nach Anspruch 1, wobei das Modell zur Bestimmung der Funktionsparameter ein Funktionsmodell reduzierter Anordnung ist.

10. Verfahren zur Bestimmung eines physiologischen Funktionsparameters eines Lebewesens (2), wobei das Verfahren Folgendes umfasst:
Bereitstellung (101) von Ultraschallbilddaten und Dopplerbilddaten einer Gefäßstruktur des Lebewesens (2) mit einer Bildbereitstellungseinheit (11);
Registrierung (102) der Ultraschallbilddaten und der Dopplerbilddaten in zeitlicher und räumlicher Hinsicht, wodurch registrierte Bilddaten erhalten werden;
Segmentierung (103) der Gefäßstruktur in den registrierten Bilddaten;
Erzeugen (104) einer Darstellung (200) der Gefäßstruktur auf der Grundlage der segmentierten Gefäßstruktur, wodurch eine Segmentierung der Gefäßstruktur erzeugt wird;
Bestimmung (105) von Strömungsgeschwindigkeitswerten in einem Gefäß der Gefäßstruktur auf der Grundlage der Dopplerbilddaten;
Bereitstellen (106) eines funktionalen Parameterbestimmungsmodells (210), das einen funktionalen physiologischen Parameter in Abhängigkeit von einer Darstellung einer Gefäßstruktur und Strömungsgeschwindigkeitswerten innerhalb eines Gefäßes der Gefäßstruktur definiert;
Bestimmen (107) der physiologischen Funktionsparameter des Lebewesens (2) unter Verwendung des Funktionsparameter-Bestimmungsmodells (210), der erzeugten Darstellung der Gefäßstruktur und der ermittelten Strömungsgeschwindigkeitswerte.

11. Verfahren nach Anspruch 10, wobei die Dopplerbilddaten erste Dopplerbilddaten eines ersten Abschnitts der Gefäßstruktur sind und die bestimmten Strömungsgeschwindigkeitswerte erste Strömungsgeschwindigkeitswerte innerhalb eines Gefäßes des ersten Abschnitts der Gefäßstruktur sind, wobei das Verfahren ferner Folgendes umfasst:
Bereitstellen (101) zweiter Doppler-Bilddaten eines zweiten Abschnitts der
Gefäßstruktur;
Registrieren (102) der zweiten Dopplerbilddaten mit den Ultraschallbilddaten;
Bestimmen (104) zweiter Strömungsgeschwindigkeitswerte innerhalb eines Gefäßes des zweiten Abschnitts der Gefäßstruktur auf der Grundlage der zweiten Dopplerbilddaten; und
Bestimmung (105) des physiologischen Funktionsparameters unter Verwendung des bereitgestellten Modells (210), der erzeugten Darstellung der Gefäßstruktur und der ersten und zweiten Strömungsgeschwindigkeitswerte.

12. Verfahren nach Anspruch 10, wobei die Ultraschallbilddaten erste Ultraschallbilddaten sind, die einen ersten Teil der Gefäßstruktur abdecken, wobei die erzeugte Gefäßstruktursegmentierung eine erste Gefäßstruktursegmentierung ist und wobei das Verfahren ferner Folgendes umfasst:
Bereitstellen zweiter Ultraschallbilddaten der Gefäßstruktur, die einen zweiten Teil der Gefäßstruktur abdecken, wobei sich der zweite Teil zumindest teilweise von dem ersten Teil unterscheidet;
Segmentierung der Gefäßstruktur in den zweiten Ultraschallbilddaten, wodurch eine zweite Gefäßstruktursegmentierung erzeugt wird;
Erzeugen der Darstellung (200) der Gefäßstruktur auf der Grundlage der ersten Gefäßstruktursegmentierung und der zweiten Gefäßstruktursegmentierung.

13. Computerprogramm zur Bestimmung eines physiologischen Funktionsparameters eines Lebewesens (2), das in einer Verarbeitungseinheit eines Apparates (10), wie in Anspruch 1 definiert, ausführbar ist, wobei das Computerprogramm Programmcodemittel umfasst, um die Verarbeitungseinheit zu veranlassen, ein Verfahren, wie in Anspruch 10 definiert, auszuführen, wenn das Computerprogramm in der Verarbeitungseinheit ausgeführt wird.

## Revendications

1. Appareil (10) pour déterminer un paramètre fonctionnel physiologique d'un être vivant (2), l'appareil (10) comprenant:
une unité de fourniture d'image (11) pour fournir des données d'image ultrasonore et des données d'image Doppler d'une structure de vaisseau de l'être vivant (2) ;
une unité d'enregistrement (12) configurée pour aligner temporellement et spatialement les données d'image ultrasonore et les données d'image Doppler pour enregistrer les données d'image ultrasonore et les données d'image Doppler afin d'obtenir des données d'image enregistrées;
une unité de segmentation (13) pour segmenter la structure du vaisseau dans les données d'image enregistrées, générant ainsi une segmentation de la structure du vaisseau;
une unité de génération de représentation (14) pour générer une représentation (200) de la structure du vaisseau basée sur la segmentation de la structure du vaisseau;
une unité de détermination de vitesse de débit (15) pour déterminer des valeurs de vitesse de débit à l'intérieur d'un vaisseau de la structure du vaisseau sur la base des données d'image Doppler; et
une unité de détermination de paramètre fonctionnel physiologique (16) pour déterminer le paramètre fonctionnel physiologique de l'être vivant (2), dans laquelle l'unité de détermination de paramètre fonctionnel physiologique est adaptée pour a) fournir un modèle de détermination de paramètre fonctionnel (210) définissant un paramètre physiologique fonctionnel en fonction d'une représentation d'une structure de vaisseau et de valeurs de vitesse de débit à l'intérieur d'un vaisseau de la structure de vaisseau, et b) déterminer le paramètre fonctionnel physiologique en utilisant le modèle de détermination de paramètre fonctionnel, la représentation générée de la structure de vaisseau et les valeurs de vitesse de débit déterminées.

2. Appareil (10) selon la revendication 1, dans lequel les données d'image Doppler sont des premières données d'image Doppler d'une première partie de la structure de vaisseau et les valeurs de vitesse de débit déterminées sont des premières valeurs de vitesse de débit à l'intérieur d'un vaisseau de la première partie de la structure de vaisseau;
l'unité de fourniture d'image (11) est configurée pour fournir des deuxièmes données d'image Doppler d'une deuxième partie de la structure de vaisseau;
l'unité d'enregistrement (12) est configurée pour enregistrer les deuxièmes données d'image Doppler avec les données d'image ultrasonore;
l'unité de détermination de valeur de vitesse de débit (15) est configurée pour déterminer des deuxièmes valeurs de vitesse de débit à l'intérieur d'un vaisseau de la deuxième partie de la structure de vaisseau sur la base des deuxièmes données d'image Doppler; et
l'unité de détermination de paramètre fonctionnel physiologique (16) est configurée pour déterminer le paramètre fonctionnel physiologique en utilisant le modèle de détermination de paramètre fonctionnel fourni (210), la représentation générée de la structure du vaisseau et les première et deuxième valeurs de vitesse de débit déterminées.

3. Appareil (10) selon la revendication 1, dans lequel les données d'images ultrasonores sont des premières données d'image ultrasonore couvrant une première partie de la structure du vaisseau, dans lequel la segmentation de la structure du vaisseau générée est une première segmentation de la structure du vaisseau et dans lequel l'unité de fourniture d'images (11) est en outre configurée pour fournir des deuxièmes données d'image ultrasonore de la structure du vaisseau couvrant une deuxième partie de la structure du vaisseau, dans lequel la deuxième partie diffère au moins partiellement de la première partie;
l'unité d'enregistrement (12) est configurée pour enregistrer les deuxièmes données d'image ultrasonore avec les premières données d'image ultrasonore;
l'unité de segmentation (13) est configurée pour segmenter la structure du vaisseau dans les deuxièmes données d'image ultrasonore, générant ainsi une deuxième segmentation de la structure du vaisseau;
l'unité de génération de représentation (14) est configurée pour générer la représentation (200) de la structure du vaisseau sur la base de la première segmentation de la structure du vaisseau et sur la base de la deuxième segmentation de la structure du vaisseau.

4. Appareil (10) selon la revendication 1, dans lequel l'unité d'enregistrement (12) comprend une unité de détection de position fournissant des données de position d'une sonde à ultrasons (1) utilisée pour générer les données d'image ultrasonore et les données d'image Doppler, dans lequel l'unité d'enregistrement (12) est adaptée pour utiliser les données de position pour enregistrer les données d'image ultrasonore et les données d'image Doppler.

5. Appareil (10) selon la revendication 1, dans lequel l'unité de segmentation (13) est configurée pour identifier des zones mobiles et statiques dans les données d'image Doppler et pour segmenter la structure du vaisseau en se basant au moins en partie sur les zones mobiles et statiques identifiées.

6. Appareil (10) selon la revendication 5, dans lequel l'unité de segmentation (13) est configurée pour déterminer des valeurs de débit de pointe locales pour un vaisseau de la structure de vaisseau dans les zones mobiles des données d'image Doppler, pour déterminer des zones transversales du vaisseau sur la base des valeurs de débit de pointe locales et pour segmenter la structure de vaisseau dans les données d'image enregistrées en utilisant les zones transversales.

7. Appareil (10) selon la revendication 5, dans lequel l'unité de segmentation (13) est en outre configurée pour segmenter la structure de vaisseau dans les données d'image enregistrées en utilisant un algorithme de détection de bord de lumière.

8. Appareil (10) selon la revendication 1, dans lequel le paramètre fonctionnel physiologique est un gradient de pression vasculaire ou une réserve de flux fractionnaire périphérique.

9. Appareil (10) selon la revendication 1, dans lequel le modèle de détermination des paramètres fonctionnels est un modèle fonctionnel d'ordre réduit.

10. Procédé pour déterminer un paramètre fonctionnel physiologique d'un être vivant (2), le procédé comprenant:
la fourniture (101) de données d'image ultrasonore et de données d'image Doppler d'une structure de vaisseau de l'être vivant (2) avec une unité de fourniture d'image (11);
l'enregistrement (102) des données d'image ultrasonore et des données d'image Doppler dans le temps et dans l'espace, pour obtenir ainsi des données d'image enregistrées;
segmenter (103) la structure du vaisseau dans les données d'image enregistrées;
générer (104) une représentation (200) de la structure du vaisseau basée sur la structure segmentée du vaisseau, générant ainsi une segmentation de la structure du vaisseau;
déterminer (105) des valeurs de vitesse de débit à l'intérieur d'un vaisseau de la structure du vaisseau sur la base des données d'image Doppler;
fournir (106) un modèle de détermination de paramètre fonctionnel (210) définissant un paramètre physiologique fonctionnel en fonction d'une représentation d'une structure de vaisseau et de valeurs de vitesse de débit à l'intérieur d'un vaisseau de la structure de vaisseau;
déterminer (107) le paramètre physiologique fonctionnel de l'être vivant (2) en utilisant le modèle de détermination de paramètre fonctionnel (210), la représentation générée de la structure du vaisseau et les valeurs de vitesse de débit déterminées.

11. Procédé selon la revendication 10, dans lequel les données d'image Doppler sont des premières données d'image Doppler d'une première partie de la structure du vaisseau et les valeurs de vitesse de débit déterminées sont des premières valeurs de vitesse de débit à l'intérieur d'un vaisseau de la première partie de la structure du vaisseau, le procédé comprenant en outre:
la fourniture (101) de deuxièmes données d'image Doppler d'une deuxième partie de la structure de vaisseau;
l'enregistrement (102) des deuxièmes données d'image Doppler avec les données d'image ultrasonore;
déterminer (104) des deuxièmes valeurs de vitesse de débit à l'intérieur d'un vaisseau de la deuxième partie de la structure du vaisseau sur la base des deuxièmes données d'image Doppler; et
déterminer (105) le paramètre fonctionnel physiologique en utilisant le modèle fourni (210), la représentation générée de la structure du vaisseau et les premières et deuxièmes valeurs de vitesse de débit.

12. Procédé selon la revendication 10, dans lequel les données d'image ultrasonore sont des premières données d'image ultrasonore couvrant une première partie de la structure du vaisseau, dans lequel la segmentation de la structure du vaisseau générée est une première segmentation de la structure du vaisseau et dans lequel le procédé comprend en outre:
la fourniture de deuxièmes données d'image ultrasonore de la structure du vaisseau couvrant une deuxième partie de la structure du vaisseau, dans laquelle la deuxième partie diffère au moins partiellement de la première partie;
segmenter la structure du vaisseau dans les deuxièmes données d'image ultrasonore, générant ainsi une deuxième segmentation de la structure du vaisseau;
générer la représentation (200) de la structure du vaisseau basée sur la première segmentation de la structure du vaisseau et la deuxième segmentation de la structure du vaisseau.

13. Programme informatique pour déterminer un paramètre fonctionnel physiologique d'un être vivant (2) exécutable dans une unité de traitement d'un appareil (10) tel que défini dans la revendication 1, le programme informatique comprenant un moyen de code de programme pour amener l'unité de traitement à exécuter un procédé tel que défini dans la revendication 10 lorsque le programme informatique est exécuté dans l'unité de traitement.
